Europäisches Patentamt

European Patent Office

Office européen des brevets

(11) Publication number: **0 513 789 A2**

## EUROPEAN PATENT APPLICATION

(21) Application number: **92108157.6**

(22) Date of filing: **14.05.92**

(51) Int. Cl.5: **G01N 33/48, A61M 1/00**

(30) Priority: **16.05.91 JP 139503/91**

(43) Date of publication of application:
**19.11.92 Bulletin 92/47**

(84) Designated Contracting States:
**DE FR GB**

(71) Applicant: **NEC CORPORATION**
**7-1, Shiba 5-chome Minato-ku**
**Tokyo 108-01(JP)**

(72) Inventor: **Ito, Narushi, c/o NEC Corporation**
**7-1, Shiba 5-chome, Minato-ku**
**Tokyo(JP)**

(74) Representative: **Pätzold, Herbert, Dr.-Ing.**
**Steubstrasse 10**
**W-8032 Gräfelfing(DE)**

(54) Method and apparatus for detecting and determining components of body fluids.

(57) In a method for determining components of a body fluid and an apparatus for carrying out the method, a body fluid-sampling cell comes in close contact with the skin of the human body and the body fluid is sampled from the skin by aspirating, under negative pressure, the skin through an aspiration port with a suction pump. A housing is positioned over a body fluid-sampling path which communicates the sampling cell to the suction pump and a quantity measuring valve arranged therein has a through hole which simultaneously serves as a reservoir for the body fluid. The through hole of the valve is rotated by a controller, then the constant amount of the sampled body fluid is forced into a flow type measuring cell through the measuring path by a liquid supply pump and the components of the body fluid are detected and determined by a sensor arranged within the measuring cell. The apparatus and method makes it possible to precisely determine the concentration of a specific component present in a very small amount of the body fluid and to continuously monitor the concentration.

EP 0 513 789 A2

## BACKGROUND OF THE INVENTION

### 1. Field of the Invention

The present invention relates to a method for detecting and determining components of a body fluid and an apparatus for carrying out the method and more particularly to a method and an apparatus for detecting and determining components of a body fluid in which only a very small amount of the body fluid is utilized.

### 2. Description of the Prior Art

Until now, the determination of biologically related compounds has been carried out by sampling blood from a subject and analyzing the blood by a clinical test apparatus. However, the sampling of the blood requires several operations for the separation of desired components thereof and accordingly there is a danger of invasion or infection.

Recently, there has been reported an application of aspiration exudate to the determination of biologically related compounds (see, for instance, Proceedings of the First Pan Pacific Symposium, Vancouver, Canada, July 23-27, 1986, pp.57-58; Proceedings of the Symposium on Chemical Sensors, PV89-9, pp.327-333; and Sensors and Actuators, B1, 1990, pp.488-490). The term "aspiration exudate" means a very small amount of liquid obtained by aspirating, under negative pressure, the portion of the skin from which the corneum is removed and is considered to be an extracellular fluid (e.g. interstitial fluid) of the hypoderm or a liquid obtained by filtration under negative pressure through the capillary wall. The aspiration exudate has a protein content lower than that of the blood, but comprises biologically related compounds having a relatively low molecular weight in amounts almost comparable to those for the blood. The aspiration exudate is rather preferable for the determination of such biologically related compounds as compared with the blood which is required for the removal of hemocytes. In addition, the aspiration exudate which is transcutaneously collected is likewise preferred for the purpose of preventing any infection of the subject.

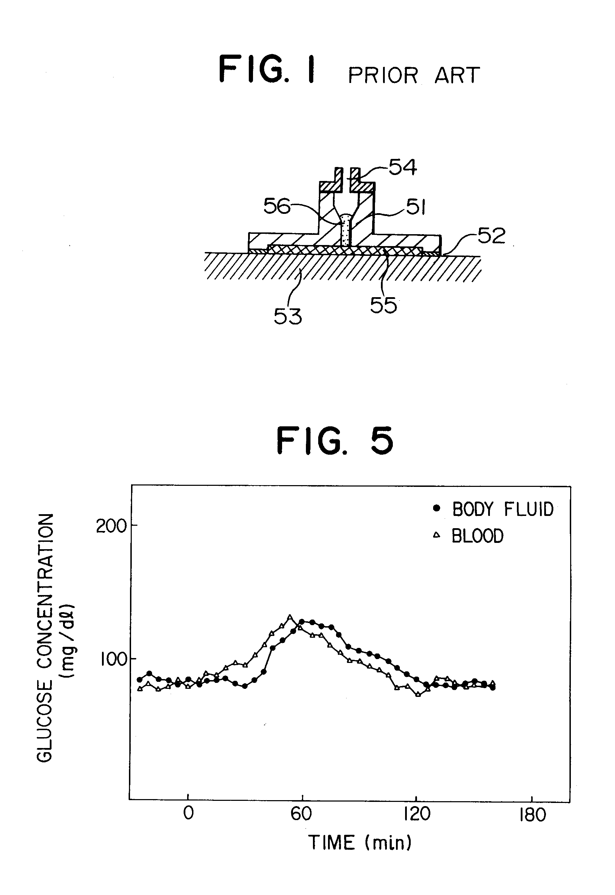

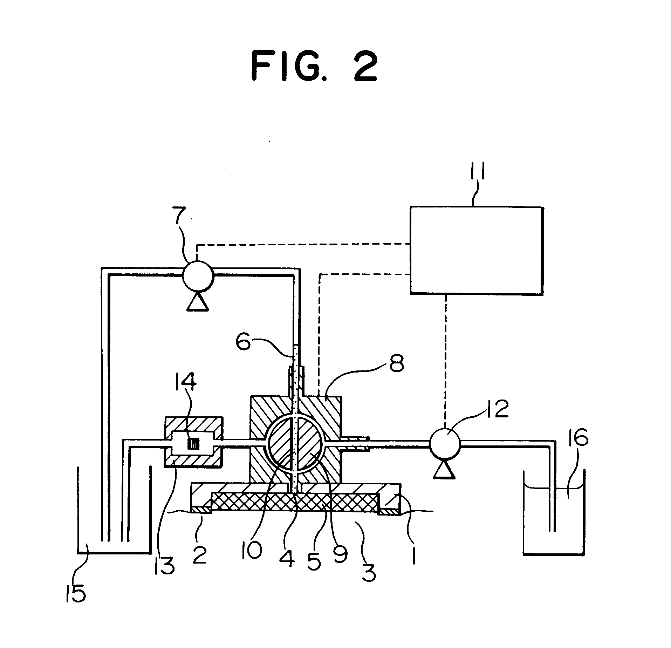

FIG. 1 is a sectional view of an embodiment of the conventional apparatus for sampling aspiration exudates. In this figure, a cell 51 for sampling a body fluid comes in close contact with the skin 53 by means of an adhesive tape 52. The body fluid 56 is sampled from the skin 53 through a spacer 55 by aspirating, under negative pressure, the skin through an aspiration port 54.

In the conventional apparatus for sampling the exudate, however, it is necessary to transfer collected samples to an analyzer for every measurements. This requires a great deal of man power and results in the sampling of an undesirably large amount of the body fluid. In particular, when it is intended to monitor any change in a specific component of the body fluid with time, the foregoing drawbacks result in giving a subject unnecessary pains and physical emaciation of the subject.

## SUMMARY OF THE INVENTION

It is in general an object of the present invention to solve the foregoing problems associated with the conventional techniques and more specifically to provide a method for detecting and determining components of a body fluid, which permits the determination while using a very small volume of a body fluid sample and which can continuously monitor a specific component thereof.

It is another object of the present invention to provide an apparatus for carrying out the foregoing method for detecting and determining components of a body fluid.

According to one aspect of the present invention, there is provided a method for determining components of a body fluid which comprises making a body fluid sampling cell and the skin of a subject to be examined come closely into contact, sampling the body fluid through aspiration under negative pressure by a suction pump communicated to an aspiration port of the sampling cell and analyzing the sampled fluid, wherein the method comprises the steps of sampling and dispensing a predetermined small volume of the body fluid derived from the skin, forcing the sampled and dispensed body fluid into a flow type measuring cell using a diluent, determining the concentration of components of the body fluid by a body fluid component detection sensor arranged within the flow type measuring cell and allowing a diluent to flow through the flow type measuring cell to discharge the measured fluid out of the flow type measuring cell, and wherein the foregoing steps are repeated in order over desired times.

According to another aspect of the present invention, there is provided an apparatus for carrying out the foregoing method which comprises a body fluid sampling cell for collecting a body fluid derived from the skin of a subject to be examined, a means for sampling, dispensing and storing the body fluid, a suction pump for reducing the pressure in the body fluid sampling cell during sampling, a flow type measuring cell equipped with a sensor for detecting the concentration of components of the body fluid, a liquid supply pump for feeding a diluent to the flow type measuring cell through the means for sampling, dispensing and storing the body fluid, a means for feeding the sampled and dispensed body fluid to the flow type measuring cell, and an exhaust port for discharging the liquid which passes through the flow type measuring cell out of the measuring system.

## BRIEF DESCRIPTION OF THE DRAWINGS

FIG. 1 is a longitudinal sectional view of an embodiment of the conventional apparatus for sampling an exudate through aspiration;

FIG. 2 is a diagram showing the structure of an embodiment of the apparatus for determining components of the body fluid according to the present invention;

FIG. 3 is a diagram showing the structure of another embodiment of the apparatus for determining components of the body fluid according to the present invention;

FIG. 4 is a diagram showing the structure of a still another embodiment of the apparatus for determining components of the body fluid according to the present invention; and

FIG. 5 is a graph showing an example of the result obtained using the apparatus according to the present invention.

## DESCRIPTION OF THE PREFERRED EMBODIMENTS

According to a first embodiment of the present invention, there is provided a method for determining components of a body fluid which comprises making a body fluid sampling cell and the skin of a subject to be examined come closely into contact, sampling the body fluid through aspiration under negative pressure by a suction pump communicated to an aspiration port of the sampling cell and analyzing the sampled body fluid, wherein the method comprises the steps of filling a through hole of a quantity measuring (or dispensing) valve positioned in the sampling path with the body fluid derived from the skin, rotating the quantity measuring valve and forcing the fluid in the through hole into a flow type measuring cell using a diluent, determining the concentration of components of the body fluid by a body fluid component detection sensor disposed in the flow type measuring cell and allowing a diluent to flow through the flow type measuring cell to discharge the measured fluid out of the flow type measuring cell, and wherein the foregoing steps are repeated in order over desired times.

According to a second embodiment of the present invention, there is provided a method for determining components of a body fluid as described in the foregoing first embodiment, except that the filling step is replaced by the step of filling a quantity measuring flow path positioned in the sampling path with the body fluid derived from the skin and the rotating and forcing steps are replaced by the steps of switching the quantity measuring flow path and forcing the fluid within the quantity measuring flow path into the flow type measuring cell using the diluent.

According to a third embodiment of the present invention, there is provided a method for determining components of a body fluid as described in the foregoing first embodiment, except that the filling step is replaced by the step of filling a well or reservoir having a constant volume positioned in the body fluid sampling cell with the body fluid derived from the skin and the rotating and forcing steps are replaced by the step of forcing the fluid within the well into the flow type measuring cell using the diluent.

According to a fourth embodiment of the present invention, there is provided an apparatus which comprises a body fluid sampling cell for collecting a body fluid derived from the skin of a subject to be examined, a suction pump for reducing the pressure in the body fluid sampling cell, a liquid supply pump for feeding a diluent, a flow type measuring cell equipped with a sensor for detecting the concentration of components of the body fluid, a quantity measuring valve for quantifying the sampled body fluid and for switching from the flow path between the sampling cell and the suction pump to that between the liquid supply pump and the measuring cell or vice versa, and an exhaust port for discharging the liquid which passes through the measuring cell out of the measuring system.

According to a fifth embodiment of the present invention, there is provided an apparatus as described in the foregoing fourth embodiment, except that the quantity measuring valve is replaced by a combination of a flow path for quantifying the sampled body fluid and a valve for switching the flow path from the connection between the sampling cell and the suction pump to that between the liquid supply pump and the measuring cell or vice versa.

According to a sixth embodiment of the present invention, there is provided an apparatus as described in the foregoing fourth embodiment, except that the body fluid sampling cell is further equipped with a well or reservoir for reserving and quantifying the sampled body fluid and the quantity measuring valve is replaced by a combination of the well and valves for opening and closing the flow path between the sampling cell and the suction pump and that between the liquid supply pump and the measuring cell, respectively.

According to the present invnetion, there is provided an apparatus for determining components (such as urea, glucose, lactic acid, creatinine and electrolytes) present in body fluids such as blood, aspiration exudates obtained through aspiration, under negative pressure, of the skin from which the corneum has been removed and the sweat.

The apparatus is provided with a well or reservoir capable of sampling and dispensing a constant volume of the body fluid in the sampling path, which may be a quantifying well or flow path or a through hole of a valve. The body fluid is first sampled in the well. The well is then communicated to the flow type measuring cell and a diluent having a constant pH and pH buffer capacity and containing a sufficient quantity of dissolved oxygen is introduced into the well to thus guide a constant amount of the body fluid present in the well together with the diluent to the flow type measuring cell. The flow type measuring cell is equipped with sensors for detecting components of the body fluid, such as urea and glucose, therein and the sensors output peak-like signals upon passage of the body fluid through the sensors at a constant flow rate. Thus, the concentrations of the components of the body fluid are highly precisely determined by every desired sensors. In this respect, the precision of the sensor outputs varies depending on the volume of the body fluid dispensing portion and the flow rate of the body fluid passing through the flow type measuring cell.

In the method for determining components of the body fluid according to the present invention, there is used a diluent having a constant pH, pH buffer capacity and ionic strength and containing a sufficient quantity of dissolved oxygen. Therefore, the initial conditions for the measurement by the sensors can be controlled and if there is used, as a sensor, a glucose sensor comprising a combination of a membrane on which glucose oxidase are immobilized and an ion sensitive field effect transistor (hereinafter referred to as "ISFET" for simplicity), glucose is rapidly oxidized due to the presence of the glucose oxidase and simultaneously any pH change in proportion to an increase or decrease of the glucose concentration can be detected by the ISFET. This results in the substantial improvement in the accuracy of the measurement.

Moreover, the repetition of the foregoing steps in order permits semi-continuous determination of a specific component of the body fluid and the monitoring of the component using only a small volume of the body fluid.

Non-limitative specific embodiments of the present invention will hereinafter be explained in more detail while referring to the attached drawings.

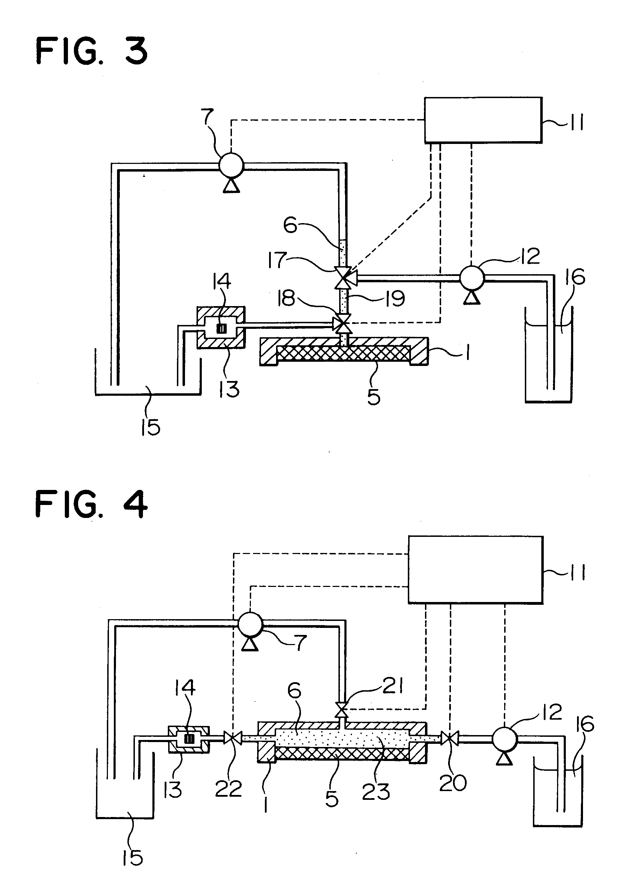

FIG. 2 is a diagram showing the structure or construction of an embodiment of the apparatus for determining components of the body fluid according to the present invention. This apparatus corresponds to the foregoing fourth embodiment of the present invention.

In FIG. 2, a body fluid-sampling cell 1 comes in close contact with the skin 3 of a subject such as the human body by means of an adhesive tape 2. The body fluid 6 is sampled from the skin 3 through a spacer 5 by aspirating, under negative pressure, the skin through an aspiration port 4 like the conventional aspiration exudate-sampling apparatus. In this embodiment, a housing 8 is positioned over a body fluid-sampling path which communicates the sampling cell 1 to a suction pump 7 and a quantity measuring valve 9 arranged therein has a through hole 10 which simultaneously serves as a reservoir for the body fluid 6. The quantity measuring valve 9 is controlled by a controller 11. Upon determination, the valve 9 is controlled to communicate the sampling path to the suction pump 7 to collect the body fluid sample so as to completely fill the through hole thereof. Thus, a desired amount of the body fluid can be sampled since the through hole 10 also serves as a dispensing means. The valve 9 is then rotated to connect the through hole 10 to the measuring path which communicates a liquid supply pump 12 and a flow type measuring cell 13. The constant amount of the body fluid 6 is forced into the measuring cell 13 through the measuring path by operating the liuqid supply pump 12, the components of the body fluid are detected by a sensor 14 arranged within the measuring cell 13 while the body fluid flows through the measuring cell 13 and the measured body fluid is discharged into a drain 15.

The apparatus according to this embodiment can be used to determine, for instance, the concentration of glucose present in a body fluid, for instance, an exudate. The procedures therefor will be detailed below.

In FIG. 2, the through hole 10 of the quantity measuring valve 9 is first adjusted to keep the horizontal state, then a 20 mM Hydroxyethylpiperazine-N'-ethanesulfonic acid (HEPES) buffered solution having a pH of 7.5 as a diluent 16 is introduced by the operation of the liquid supply pump 12 according to the directions of the controller 11 to fill the measuring path, the through hole 10 of the quantity measuring valve 9 and the flow type measuring cell 13 with the diluent 16.

Then the quantity measuring valve 9 is rotated at an angle of 90° by the controller 11 so that the through hole 10 of the valve 9 is connected to the sampling path which communicates the body fluid-sampling cell 1 and the suction pump 7, then the suction pump 7 is driven by the controller 11 to fill the through hole 10 with the body fluid 6.

Thereafter, the through hole 10 of the valve 9 is again rotated at an angle of 90° according to the directions of the controller 11, then the liquid supply pump 12 is operated to force the sampled body fluid 6 having a predetermined constant volume into the measuring cell 13 by the action of the diluent 16 pumped by the supply pump 12 at a constant flow rate. At this stage, the sampled body fluid passes through the measuring cell 13 at a constant flow rate and the concentration of glucose present therein is detected and determined by the sensor 14 arranged within the measuring cell 13. The foregoing steps can be, in order, repeated to perform semi-continuous determination or monitoring of the glucose concentration through the control of the controller 11.

FIG. 3 is a diagram showing the structure of another embodiment of the apparatus for determining components of the body fluid according to the present invention. In FIGS. 3 and 4 as will be detailed below, all the parts and/or elements which are identical to those depicted in FIG. 2 bear the same reference numerals and details thereof are thus omitted for simplicity.

This apparatus of FIG. 3 corresponds to the foregoing fifth embodiment of the present invention and comprises two three-way valves 17 and 18 positioned in the course of the body fluid-sampling path which communicates the body fluid-sampling cell 1 to the suction pump 7 and in this embodiment, a flow path 19 defined by and positioned between these two three-way valves 17 and 18 serves as a reservoir as well as a dispensing and sampling means for the body fluid 6. The three-way valves 17 and 18 are controlled and switched by the controller 11 so that they communicate the flow path 19 to the flow type measuring cell 13 after sampling and dispensing the body fluid. The dispensed body fluid 6 is forced into the measuring cell 13 by operating the liquid supply pump 12, then the concentration of the desired component of the body fluid is determined by the sensor 14 arranged within the measuring cell 13 while the dispensed body fluid passes through the measuring cell at a constant flow rate and finally the measured sample is discharged into the drain 15.

The apparatus according to the fifth embodiment of the present invention can likewise be used to determine, for instance, the concentration of glucose present in a body fluid, for instance, an exudate. The procedures therefor will be detailed below.

The three-way valves 17 and 18 are adjusted by the controller 11 so that they communicate the measuring cell 13 and the liquid supply pump 12 while intercepting the path communicated to the suction pump 7 and that communicated to the sampling cell 1, respectively, then the supply pump 12 is operated and a buffered solution, for instance, a 20 mM HEPES buffered solution having a pH of 7.5, as a diluent 16 is introduced into the measuring path according to the directions of the controller 11 to fill the measuring path, the flow path 19 for dispensing the body fluid and the flow type measuring cell 13 with the diluent 16.

Thereafter, the three-way valve 17 is controlled by the controller 11 so that the path communicated to the liquid supply pump 12 is cut off while opening the path communicated to the suction pump 7. On the other hand, the three-way valve 18 is likewise controlled by the controller 11 so that the path communicated to the sampling cell 1 is opened and that communicated to the flow type measuring cell 13 is closed. Then the suction pump 7 is driven by the controller 11 to thus fill the quantity measuring path 19 with the body fluid 6.

Further, the three-way valve 17 is adjusted by the controller 11 so that the path communicated to the liquid supply pump 12 is opened while the path communicated to the suction pump 7 is closed. On the other hand, the three-way valve 18 is likewise adjusted so as to cut off the path communicated to the sampling cell 1 and to open the path communicated to the measuring cell 13. The diluent 16 is fed to the flow path 19 to thus force the dispensed body fluid 6 into the measuring cell 13. In the measuring cell 13, the sensor 14 positioned therein detects glucose present in the body fluid and determines the concentration thereof while the fluid passes through the measuring cell 13 at a constant flow rate. The foregoing steps can be, in order, repeated to perform semi-continuous determination or monitoring of the glucose concentration through the control of the controller 11.

FIG. 4 is a diagram showing the structure of a further embodiment of the apparatus for determining components of the body fluid according to the present invention. This apparatus corresponds to the sixth embodiment of the present invention as mentioned above and comprises a first valve 20 positioned in the course of a path which communicates the body fluid-sampling cell 1 to the liquid supply pump 12, a second valve 21 positioned between the sampling cell 1 and the suction pump 7, a third valve 22 positioned between the sampling cell 1 and the flow type measuring cell 13, and a liquid reservoir 23 for the sampled body fluid 6 which is formed within the sampling cell 1 and simultaneously serves as a quantity measuring means. The flow paths are switched by the valves which are controlled by the controller 11 so that the liquid supply pump 12, the flow type measuring cell 13 and the liquid reservoir 23 are intercommunicated to one another while cutting off the flow path connected to the suction pump 7 or that the suction pump 7 and the liquid reservoir 23 are intercommunicated while the paths connected to the measuring cell 13 and the liquid supply pump 12 respectively are cut off. In this manner, the dispensed body fluid is fed to the flow type measuring cell 13 by the action of the liquid supply pump 12, and the desired components thereof are detected and determined by the sensor 14 arranged within the measuring cell 13 and the measured body fluid 6 is discharged into the drain 15.

The apparatus according to the sixth embodiment of the present invention can likewise be used to determine, for instance, the concentration of glucose present in a body fluid, for instance, an exudate. The procedures therefor will be detailed below.

First the controller 11 is operated so that the valve 21 is closed, then the liquid supply pump 12 is driven and a buffered solution, for instance, a 20 mM HEPES buffered solution having a pH of 7.5, as a diluent 16 is fed to the sampling cell 1 to thus fill the measuring cell 13 and the sampling cell 1 inclusive of the dispensing reservoir 23.

Then the valves 20 and 22 are closed while opening the valve 21 and the suction pump 7 is started, by the controller 11 to thus fill the liquid reservoir 23 with the body fluid 6.

Thereafter, the controller 11 is operated so that the valve 21 is closed while the valves 20 and 22 are opened and the liquid supply pump 12 is driven to feed the diluent 16 to the sampling cell 1 or reservoir 23 to thus force the dispensed body fluid 6 in the reservoir 23 into the measuring cell 13. At this stage, the sensor 14 arranged within the measuring cell 13 detects glucose present in the dispensed body fluid and determines the concentration thereof while the body fluid 6 passes through the measuring cell 13 at a predetermined flow rate. The foregoing steps can be, in order, repeated to perform semi-continuous determination or monitoring of the glucose concentration through the control of the controller 11.

The apparatuses discussed above are properly selected depending on the accuracy of measurement required for each component of the body fluid to be detected and determined and the manner of use selected from the clinical standpoint. The apparatus having the structure shown in FIG. 2 comprises the quantity measuring valve 9 and the driving means therefor in the housing 8 and the body fluid-sampling cell 1 which comes in contact with the skin and, therefore, ensures a high accuracy of measurement, but the apparatus limits the degree of freedom of the subject to be examined. For this reason, it is very suitable for the determination of components requiring a high accuracy which is performed near a bed. For instance, components such as $Na^+$ and $Cl^-$ in general show physiological variations falling within very narrow ranges and requires an accuracy to at most 1%. The apparatus shown in FIG. 2 can be used for the determination of these components.

In the apparatus having the structure shown in FIG. 3, the quantity measuring flow path 19 can easily and simply be replaced with a new one. This can prevent the reduction in the accuracy of measurement due to the adsorption of components in the body fluid such as albumin to the flow path and, therefore, the apparatus is suitable for continuous measurements over a long period of time. Thus, the apparatus can effectively be applied to the continuous determination of, for instance, blood-sugar levels and the content of urea over a long time period.

The apparatus having the structure shown in FIG. 4 makes it possible to miniaturize and lighten the part fitted to the skin since the sampling cell 1 and the valves have a dispensing function. This allows an increase in the degree of freedom of the subject to be examined. The apparatus can be applied to the determination of, for intance, physiological functions and degree of fatigue during taking exercise while using a sensor for detecting lactic acid.

FIG. 5 is a graph showing the results observed when the glucose concentration in an exudate is monitored by the apparatus for determining components of the body fluid as shown in FIG. 2 and shows the glucose concentration change observed during the test in which glucose is orally administered to a subject to be examined.

When, in this apparatus, the volume of the through hole 10 of the dispensing valve 9 was designed to be 5 $\mu$l, the determination of the glucose concentration could be performed using a very small amount of the exudate in intervals of 5 minutes. In FIG. 5, an amount equal to 75g of glucose was orally administered at the time 0 and then variations in the glucose concentrations in the exudate and blood were determined. As seen from FIG. 5, about 5 minutes lag was observed between the glucose concentrations in the exudate and blood, but an adequate correlation was estimated from the results.

In addition to the foregoing embodiments, the flow type measuring cell 13 may further be provided with an inlet port for introducing a liquid containing a known component in a known concentration to thus carry out the calibration of the sensor 14.

As has been explained above in detail, the apparatus and method for determining components of the body fluid according to the present invention makes it possible to precisely determine the concentration of a specific component present in a very small amount of the body fluid and to continuously monitor the concentration.

**Claims**

1. A method for determining components of a body fluid which comprises making a body fluid sampling cell and the skin of a subject to be examined come closely into contact, sampling said body fluid through aspiration under negative pressure by a suction pump communicated to an aspiration port of said sampling cell and analyzing the sampled fluid, wherein the method comprises the steps of:

sampling and dispensing a predetermined small volume of said body fluid derived from the skin;

forcing the sampled and dispensed body fluid into a flow type measuring cell using a diluent;

determining the concentration of components of said body fluid by a body fluid component detection sensor arranged within said flow type measuring cell; and

allowing a diluent to flow through said flow type measuring cell to discharge the measured fluid out of said flow type measuring cell; and

wherein the foregoing steps are repeated in order over desired times.

2. The method for determining components of a body fluid according to claim 1 wherein said body fluid of the subject to be exained is sampled and dispensed by filling a through hole of a quantity measuring valve positioned in the sampling path with said body fluid derived from the skin and the sampled and dispensed body fluid is transferred to said flow type measuring cell by rotating said quantity measuring valve and forcing the body fluid in said through hole into said flow type measuring cell by the action of said diluent.

3. The method for determining components of a body fluid according to claim 2 wherein said component is one showing a physiological variation falling within a very narrow range.

4. The method for determining components of a body fluid according to claim 1 wherein said body fluid of the subject to be examined is sampled and dispensed by filling a quantity measuring flow path positioned and defined in the sampling path with said body fluid derived from the skin and the sampled and dispensed body fluid is transferred to said flow type measuring cell by switching said quantity measuring path and forcing the body fluid within said quantity measuring path into said flow type measuring cell by the action of said diluent.

5. The method for determining components of a body fluid according to claim 4 wherein said method is applied to a continuous measurement over a long time period.

6. The method for determining components of a body fluid according to claim 1 wherein said body fluid of the subject to be examined is sampled and dispensed by filling a well having a constant volume positioned and defined in said body fluid sampling cell with the body fluid derived from the skin and the sampled and dispensed body fluid is transferred to said flow type measuring cell by forcing the body fluid within said well into said flow type measuring cell by the action of said diluent.

7. The method for determining components of a body fluid according to claim 6 wherein said method is applied to the determination of physiological functions or degree of fatigue.

8. The method for determining components of a body fluid according to claim 1 wherein said body fluid component detection sensor is calibrated prior to the determination of the components of said body fluid by introducing a liquid containing a known component in a known concentration into said flow type measuring cell through an inlet port and determining the concentration thereof by said sensor.

9. An apparatus for determining components of a body fluid comprising:

a body fluid sampling cell for collecting a body fluid derived from the skin of a subject to be examined;

a means for sampling, dispensing and storing said body fluid;

a suction pump for reducing the pressure in said body fluid sampling cell during sampling;

a flow type measuring cell equipped with a sensor for detecting the concentration of components of said body fluid;

a liquid supply pump for feeding a diluent through said means for sampling, dispensing and storing said body fluid to said flow type measuring cell;

a means for feeding the sampled and dispensed body fluid to said flow type measuring cell; and

an exhaust port for discharging the liquid which passes through said flow type measuring cell out of the measuring system.

10. The apparatus for determining components of a body fluid according to claim 9 wherein said means for sampling, dispensing and storing the body fluid is a through hole of a valve means connected to a smapling path which communicates said body fluid sampling cell and said suction pump, and said valve means serves as said means for feeding the sampled and dispensed body fluid to said flow type measuring cell equipped with a sensor for detecting the concentration of components of said body fluid, together with said liquid supply pump.

11. The apparatus for determining components of a body fluid according to claim 10 wherein said sensor for detecting the concentration of components of the body fluid is further provided with an inlet port for introducing a liquid for calibration.

12. The apparatus for determining components of a body fluid according to claim 9 wherein said means for sampling, dispensing and storing the body fluid is a flow path positioned in the sampling path which connects said body fluid sampling cell to said suction pump and defined by a pair of valve means, and said valve means serve as said means for feeding the sampled and dispensed body fluid to said flow type measuring cell equipped with a sensor for detecting the concentration of components of said body fluid, together with said liquid supply pump.

13. The apparatus for determining components of a body fluid according to claim 12 wherein said sensor for detecting the concentration of components of the body fluid is further provided with an inlet port for introducing a liquid for calibration.

14. The apparatus for determining components of a body fluid according to claim 9 wherein said means for sampling, dispensing and storing the body fluid is a reservoir means formed within said body fluid sampling cell and defined by valve means, and said valve means serves as said means for feeding the sampled and dispensed body fluid to said flow type measuring cell equipped with a sensor for detecting the concentration of components of said body fluid, together with said liquid supply pump. 15. The apparatus of claim 14 wherein said sensor for detecting the concentration of components of the body fluid is further provided with an inlet port for introducing a liquid for calibration.

# FIG. 1   PRIOR ART

# FIG. 5

# FIG. 2

# FIG. 3

# FIG. 4